(19) Europäisches Patentamt
European Patent Office
Office européen des brevets

(11) Veröffentlichungsnummer: **0 116 329**
**A1**

(12) # EUROPÄISCHE PATENTANMELDUNG

(21) Anmeldenummer: 84100893.1

(22) Anmeldetag: 28.01.84

(51) Int. Cl.³: C 07 J 63/00
C 07 J 71/00, A 61 K 31/41
A 61 K 31/275, A 61 K 31/335

(30) Priorität: 11.02.83 CH 788/83

(43) Veröffentlichungstag der Anmeldung: 22.08.84 Patentblatt 84/34

(84) Benannte Vertragsstaaten: AT BE CH DE FR GB IT LI LU NL SE

(71) Anmelder: F. HOFFMANN-LA ROCHE & CO. Aktiengesellschaft

CH-4002 Basel(CH)

(72) Erfinder: Fürst, Andor, Dr.
Magnolienpark 14
Basel(CH)

(72) Erfinder: Müller, Marcel, Dr.
Quellenweg 10
Frenkendorf(CH)

(72) Erfinder: Kerb, Ulrich, Dr.
Prinzregentenstrasse 7
Berlin(DE)

(72) Erfinder: Wiechert, Rudolf, Prof. Dr.
Petzowerstrasse 8a
Berlin(DE)

(74) Vertreter: Grossner, Lutz, Dr. et al,
Grenzacher Strasse 124 Postfach 3255
CH-4002 Basel(CH)

(54) **Neue D-Homosteroide, deren Herstellung und pharmazeutische Präparate.**

(57) Die neuen D-Homosteroide der Formel

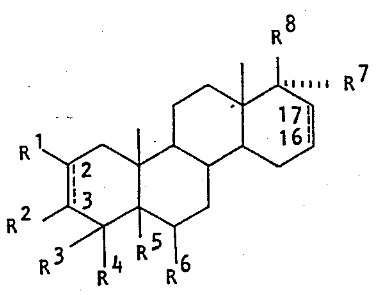

I

worin $R^1$ einen Rest –CN, =NOH oder =CH–NHOH; $R^2$ Oxo oder, falls $R^1$ ein Rest –CN ist, Oxo oder einen Rest –OAc; oder $R^1$ und $R^2$ zusammen mit den C-Atomen 2 und 3 des Steroidgerüsts einen [2,3-d]-annelierten Isoxazolring, einen [3,2-c]-annelierten Pyrazolring, einen [3,2,-c]-annelierten N-acylierten Pyrazolring oder einen [2,3-c]-annelierten Furazanring; Ac einen Acylrest; $R^3$ und $R^4$ Methyl und $R^5$ und $R^6$ gemeinsam eine zusätzliche Bindung zwischen den C-Atomen 5 und 6 des Steroidgerüsts; oder $R^4$ und $R^5$ gemeinsam –O– $R^3$ Wasserstoff oder Methyl, und $R^6$ Wasserstoff; $R^7$ Wasserstoff, nieder-Alkyl oder Aethinyl; $R^8$ Hydroxy oder Acyloxy; oder $R^7$ und $R^8$ gemeinsam einen Spiroätherrest der Formel

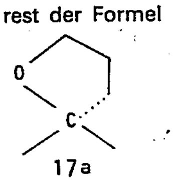

17a

oder einen Spirolactonrest der Formel

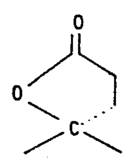

17a

darstellen, die punktierte 16,17-Bindung eine fakultative zusätzliche C-C-Bindung ist; und die punktierte 2,3-Bindung eine zusätzliche C-C-Bindung ist, wenn $R^2$ und $R^3$ zusammen mit den C-Atomen 2 und 3 den Isoxazol- oder Pyrazolring bilden oder wenn $R^2$ ein Rest –OAc ist,

hemmen die Progesteronsynthese im Organismus und können zur Fertilitätskontrolle verwendet werden. Die neuen Verbindungen können durch Molekülabwandlungen anderer D-Homosteroide hergestellt werden.

EP 0 116 329 A1

Croydon Printing Company Ltd.

0116329

F.Hoffmann-La Roche & Co. Aktiengesellschaft, Basel, Schweiz

RAN 4104/177

# Neue D-Homosteroide, deren Herstellung und pharmazeutische Präparate

Die vorliegende Erfindung betrifft neue D-Homosteroide der Formel

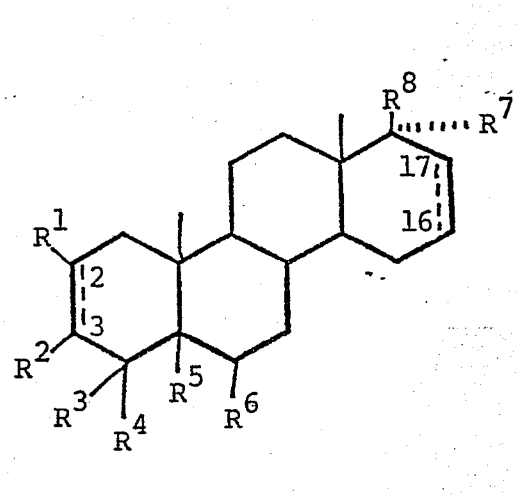

I

worin $R^1$ einen Rest -CN, =NOH oder =CH-NHOH; $R^2$ Oxo oder, falls $R^1$ ein Rest -CN ist, Oxo oder einen Rest -OAc; oder $R^1$ und $R^2$ zusammen mit den C-Atomen 2 und 3 des Steroidgerüsts einen [2,3-d]-annelierten Isoxazolring, einen [3,2-c]-annelierten Pyrazolring, einen [3,2-c]-annelierten N-acylierten Pyrazolring oder einen [2,3-c]-annelierten Furazanring; Ac einen Acylrest; $R^3$ und $R^4$ Methyl und $R^5$

Grn/ 7.12.83

und $R^6$ gemeinsam eine zusätzliche Bindung zwischen den C-Atomen 5 und 6 des Steroidgerüsts; oder $R^4$ und $R^5$ gemeinsam -O-, $R^3$ Wasserstoff oder Methyl, und $R^6$ Wasserstoff; $R^7$ Wasserstoff, nieder-Alkyl oder Aethinyl; $R^8$ Hydroxy oder Acyloxy; oder $R^7$ und $R^8$ gemeinsam einen Spiroätherrest der Formel

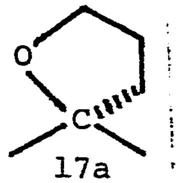

oder einen Spirolactonrest der Formel

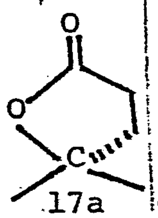

darstellen, die punktierte 16,17-Bindung eine fakultative zusätzliche C-C-Bindung ist; und die punktierte 2,3-Bindung eine zusätzliche C-C-Bindung ist, wenn $R^2$ und $R^3$ zusammen mit den C-Atomen 2 und 3 den Isoxazol- oder Pyrazolring bilden oder wenn $R^2$ ein Rest -OAc ist.

Die Erfindung betrifft weiterhin pharmazeutische Präparate, die solche D-Homosteroide als Wirkstoff enthalten; sowie ein Verfahren zur Herstellung der neuen D-Homosteroide. Die Erfindung betrifft auch die D-Homosteroide der Formel I zur Anwendung als pharmazeutische Wirkstoffe, insbesondere als Interzeptiva.

Der Ausdruck "Acyl" bezeichnet Säurereste von aliphatischen, cycloaliphatischen, aromatischen, araliphatischen und heterocyclischen Carbonsäuren. Aliphatische Carbonsäuren können gesättigt oder ungesättigt sein. Beispiele dafür sind insbesondere Alkancarbonsöuren, vorzugsweise niedere Alkancarbonsäuren wie Essigsäure, Propionsäure, Buttersäure, Pivalinsäure und Capronsäure. Beispiele von cycloaliphatischen Carbonsäuren sind Cyclopentyl- und Cyclohexylpropionsäure. Beispiele von aromatischen Carbonsäuren sind Benzoesäure und substituierte Benzoesäuren, wie p-Nitrobenzoesäure oder Toluylsäuren. Beispiele von araliphatischen Carbonsäuren sind Phenylessigsäure und Phenylpropionsäure. Beispiele von heterocyclischen Carbonsäuren sind N- oder S-heterocyclische Carbonsäuren mit vorzugsweise 5- oder 6-gliedrigem Ring wie Nicotinsäuren und Thiophencarbonsäuren. Der Ausdruck "nieder" bezeichnet Gruppen mit bis zu 6 C-Atomen. Alkylgruppen können geradkettig oder verzweigt sein. Beispiele sind Methyl, Aethyl, Propyl, Isopropyl, n-Butyl und Isomere davon.

In den Verbindungen der Formel I, in denen $R^1$ Cyano und $R^2$ Oxo darstellt, können in tautomeren Formen, nämlich in Form des 2α-Cyano-3-ketons oder in enolisierter Form als 2-Cyano-3-hydroxy-$\Delta^2$-Tautomeren vorliegen. Falls $R^4$ und $R^5$ gemeinsam Oxido (-O-) darstellen, soll die Oxidogruppe α-Konfiguration aufweisen.

Von den D-Homosteroiden der Formel I sind die 16,17-ungesättigten bevorzugt. Weiterhin sind Verbindungen der Formel I bevorzugt, in denen $R^1$ Cyano ist. Besonders bevorzugt ist das 17aβ-Hydroxy-4,4,17a-trimethyl-3-oxo-D-homoandrosta-5,16-dien-2α-carbonitril.

Die Verbindungen der Formel I können erfindungsgemäss dadurch hergestellt werden, dass man

a) ein D-Homosteroid der Formel

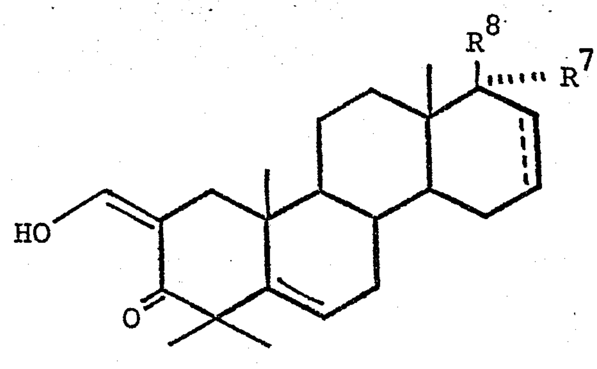

II

mit Hydroxylamin oder Hydrazin oder O,N-Bis-(trifluoracetyl)-hydroxylamin umsetzt; oder

b) ein D-Homosteroid der Formel

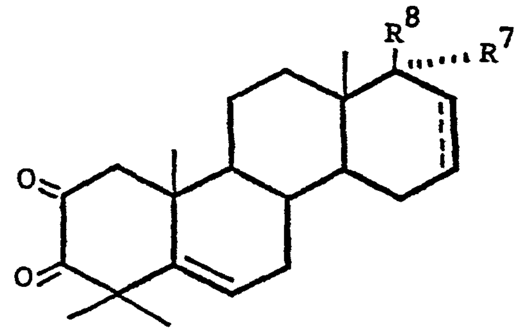

III

mit Hydroxylamin umsetzt; oder

c) ein D-Homosteroid der Formel

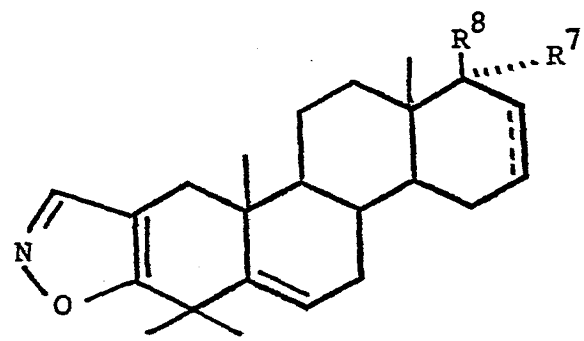

IV

mit einer starken Base behandelt; oder

d) ein D-Homosteroid der Formel

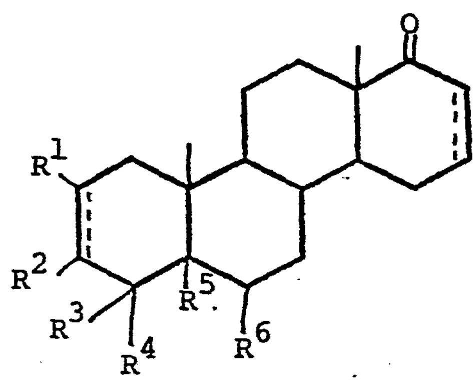

V

mit einer den Rest $R^7$ abgebenden metallorganischen Verbindung umsetzt; oder

e) ein D-Homosteroid der Formel

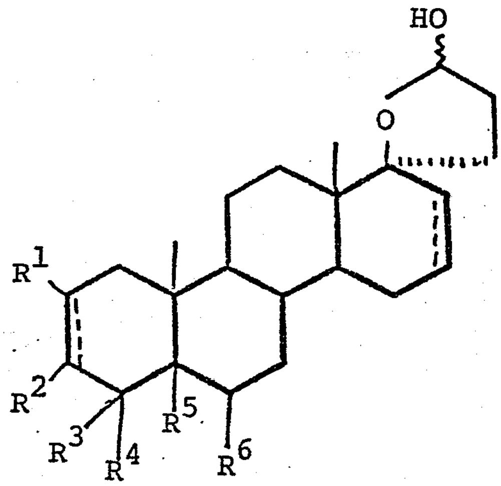

VI

mit einem Oxidationsmittel behandelt; oder

f) ein D-Homosteroid der Formel I, worin $R^1$ Cyano und $R^2$ Oxo oder $R^1$ und $R^2$ zusammen mit den C-Atomen 2 und 3 des Steroidgerüsts einen Pyrazolring darstellen und/oder $R^8$ Hydroxy ist; mit einem Acylierungsmittel behandelt, oder

g) ein D-Homosteroid der Formel

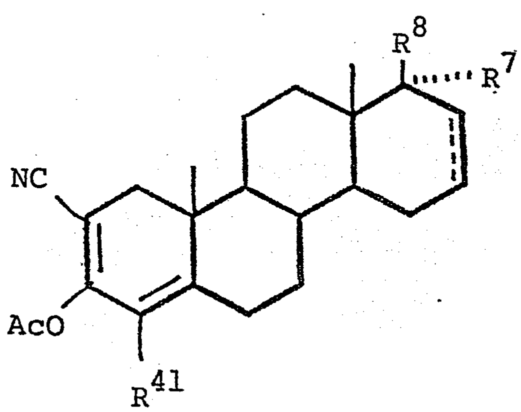

VII

mit einem Oxidationsmittel behandelt und das erhaltene 4,5-Oxid gewünschtenfalls mit einer Base behandelt, wobei in den vorstehenden Formeln II-VII Ac einen Acylrest; $R^{41}$ Wasserstoff oder Methyl darstellen und $R^1$-$R^8$ die oben angegebene Bedeutung haben.

Die Umsetzung von Verbindungen der Formel II gemäss Verfahrensvariante a) kann in an sich bekannter Weise z.B.

in organischen, inerten Lösungsmitteln, wie Aethanol oder Essigsäure unter Erwärmen vorgenommen werden. Die Umsetzung mit Hydrazin liefert Steroid [3,2-c]pyrazole der Formel

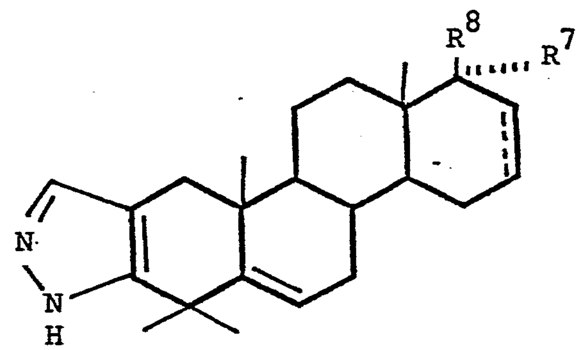

I a

worin $R^7$ und $R^8$ die obige Bedeutung haben.

Die Umsetzung mit Hydroxylamin liefert Steroid [2,3-d]isoxazole der Formel

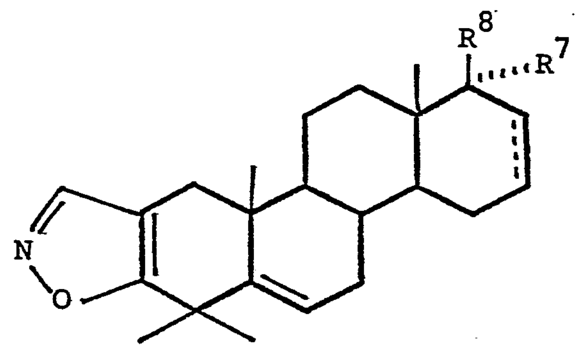

I b

worin $R^7$ und $R^8$ die obige Bedeutung haben.

Die Umsetzung mit O,N-Bis-(trifluoracetyl-hydroxylamin in Gegenwart einer Base wie Pyridin liefert 2-Cyano-Derivate der Formel

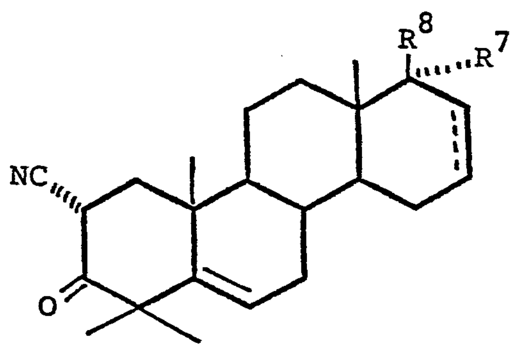

I c

worin $R^7$ und $R^8$ die obige Bedeutung haben, bzw. Enole davon.

Die Umsetzung einer Verbindung der Formel III gemäss Verfahrensvariante b) kann ebenfalls in Analogie zu der Umsetzung einer Verbindung II mit Hydroxylamin erfolgen. Man erhält dabei Steroid [2,3-c]furazane der Formel

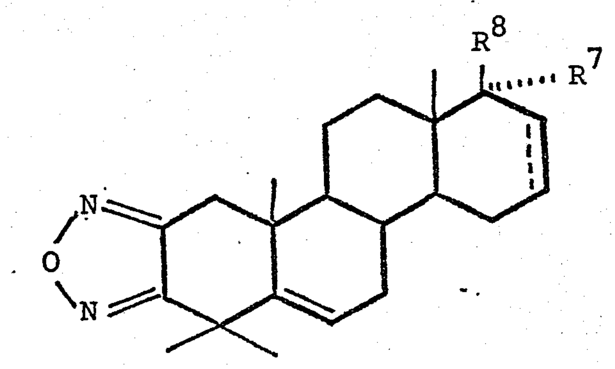

I d

worin $R^7$ und $R^8$ die obige Bedeutung haben.

Gemäss Verfahrensvariante c) wird eine Verbindung der Formel IV mit einer starken Base wie Alkalimetallalkoholaten, z.B. Natriummethylat, vorzugsweise bei Raumtemperatur behandelt. Diese Verfahrensvariante führt zu Verbindungen der Formel Ic.

Die Verfahrensvariante d) führt zu Verbindungen der Formel I in der $R^8$ Hydroxy und $R^7$ nieder-Alkyl oder Aethinyl ist. Beispiele metallorganischer Verbindungen, die mit einer Verbindung V umgesetzt werden können, sind nieder-Alkyl-Magnesiumhalogenide und Alkalimetallalkyle, wie Methyllithium und Alkalimetallacetylide wie Natrium-, Kalium- oder Lithiumacetylid. Die Umsetzung einer Verbindung V mit diesen Reagentien kann in für Umsetzungen von Carbonylverbindungen mit Grignard- und Alkalimetallorganischen Verbindungen an sich bekannter Weise vorgenommen werden.

Die Oxidation einer Verbindung VI gemäss Verfahrensvariante e) zu einer Verbindung der Formel I, in der $R^7$ und $R^8$ einen Spirolactonring darstellen, kann in an sich bekannter Weise mit Oxidationsmitteln wie Jones-Reagens ($CrO_3H_2SO_4$) bewerkstelligt werden.

Eine Verbindung der Formel Ia oder Ic kann durch Behandlung mit einem Acylierungsmittel gemäss Verfahrensvariante f) in eine Verbindung der Formeln Ie bzw. If

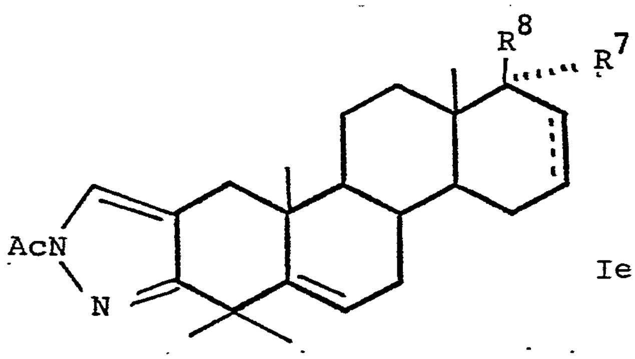

Ie

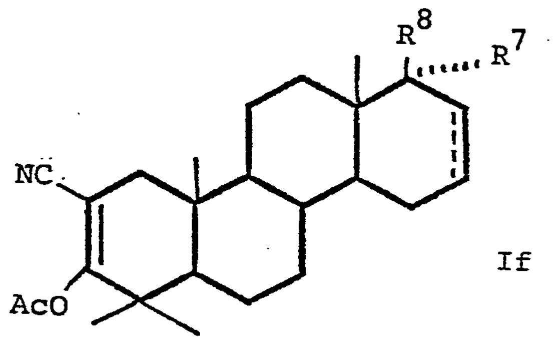

If

worin Ac Acyl ist und $R^7$ und $R^8$ die obige Bedeutung haben,

übergeführt werden.

Beispiele von Acylierungsmitteln sind Säureanhydride, z.B. nieder-Alkancarbonsäureanhydride wie Acetanhydrid und Halogenide, wie Acetylchlorid. Diese Umsetzung kann unter den für Acylierungen an sich bekannten Reaktionsbedingungen durchgeführt werden.

Die Oxidation einer Verbindung der Formel VII zu einem 4,5-Oxid (Verfahrensvariante g)) kann mit Oxidationsmitteln wie Persäuren, z.B. Perbenzoesäure oder Peressigsäure, vorgenommen werden. Man erhält so eine Verbindung der Formel

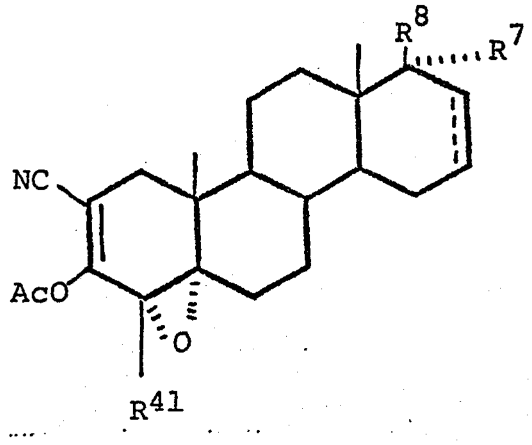

I g

worin $R^{41}$, $R^7$, $R^8$ und Ac die obige Bedeutung haben.

Behandlung einer Verbindung Ig mit Basen wie wässrigalkoholischen Alkalihydroxiden oder -carbonaten führt zur Abspaltung des Acylrests Ac und Bildung des entsprechenden 3-Ketons.

Die Verbindungen der Formel I sind pharmakologisch wirksam. Sie wirken als Inhibitoren der $\Delta^5$-Isomerase/3β-Hydroxysteroid-Dehydrogenase bzw. der körpereigenen Progesteronsynthese. Derartige Hemmstoffe führen zu einem Schwangerschaftsabbruch in frühem Stadium (Fertility and Sterility 30 (1978) 86-90).

Die Wirksamkeit der Verbindungen der Formel I als Hemmer der $\Delta^5$-Isomerase/3β-Hydroxysteroid-Dehydrogenase wurde in folgenden Versuchsanordnungen ermittelt:

in vitro:

Rattenovarien wurden in 0,05 M $Na_2HPO_4$-Puffer (pH 7,2), der 0,25 M Sucrose und 0,05 M Dithiothreit enthielt, homogenisiert. Die nach Zentrifugieren erhaltenen Mikrosomen wurden in 0,05 M $Na_2HPO_4$-Puffer (pH 7,4), der 0,001 M EDTA enthielt, suspendiert. Dieses Mikrosomenpräparat wurde zunächst auf enzymatische Aktivität durch Messung der Zunahme der optischen Dichte bei 340 nm bei der Reduktion von $NAD^+$ geprüft. Danach wurden aliquote Teile (0,5 µg Protein) 30 Minuten bei 37°C mit 250 pMol $^{14}$C-Pregnelonon, 0,4 mg $NAD^+$ und verschiedenen Konzentrationen der in mini-

0116329

malen Mengen Dimethylsulfoxid gelösten Testsubstanz in 1,8 ml Puffer inkubiert. Die Reaktion wurde durch Zusatz von Methylenchlorid abgebrochen. Nach Extraktion und Abdampfen des Lösungsmittels wurden die radioaktiven Metaboliten auf Silicagel-Aluminiumplatten mit Benzol/Aceton (85:15) getrennt. Die Radioaktivität wurde mittels Scintillationszähler gemessen.

in vivo:

Weibliche Albinoratten wurden unter kontrollierten Bedingungen gehalten, wobei die Synchronizität des Zyklus durch tägliche Vaginalspülung und nachfolgende zytologische Untersuchung überwacht wurde. Beim Proöstrus wurden die Tiere mit männlichen, fertilen Ratten zusammengebracht. Die erfolgreiche Insemination wurde am folgenden Morgen überprüft und dieser Tag als 1. Tag der Gravidität bezeichnet. Die graviden Tiere erhielten dann die Testsubstanzen als Suspension in einem Standard-Vehikel (0,5% Carboxymethylzellulose, 0,4% TWEEN 80, 0,9% Benzylalkohol und 0,9% NaCl in Wasser) oral oder intramuskulär am 10. Tag. Am 15. Tag wurden die Tiere getötet. Das Blut wurde gesammelt und die Uteri auf lebende Föten, Resorptionen und leere Implantationsstellen untersucht. Die Serum-Hormonkonzentrationen wurden radioimmunologisch bestimmt. Die Resultate sind in den nachstehenden Tabellen aufgeführt.

Tabelle 1 : Hemmung der Progesteronbildung in vitro

| Verbindung von Beispiel | $IC_{50}$ [nM] |
|---|---|
| 5 | 0,8 |
| 6 | 0,4 |
| 7 | 6 |
| 12 | 6 |
| 17 | 6,5 |
| 22 | 0,9 |
| 25 | 7 |

$IC_{50}$: Konzentration der Testverbindung, bei der die Enzymaktivität 50% des maximalen Wertes beträgt.

Tabelle 2 : Interzeptive Wirkung bei Ratten

| Verbindung von Beispiel Nr. | Dosis (mg/kg) | n | Serumprogesteron (ng/ml) 3 h später | lebende Föten | | Resorption | | leere Implantationsstellen | |
|---|---|---|---|---|---|---|---|---|---|
| | | | | n | x | n | x | n | x |
| Vehikel | - | 45 | 105 | 45 | 12 | 25 | 2 | 0 | - |
| 2 | 3,75 | 6 | 87 | 3 | 12 | 2 | 9 | 2 | 13 |
| 2 | 7,5 | 6 | 39 | 0 | - | 0 | - | 6 | 13 |
| 2 | 15 | 6 | 35 | 0 | - | 0 | - | 6 | 12 |
| 3 | 7,5 | 7 | 25 | 4 | 9 | 5 | 6 | 2 | 3 |
| 3 | 15 | 8 | 29 | 4 | 9 | 6 | 3 | 3 | 13 |
| 14 | 7,5 | 7 | 20 | 1 | 3 | 3 | 12 | 4 | 12 |
| 14 | 15 | 8 | 20 | 1 | 12 | 3 | 14 | 4 | 13 |
| 17 | 3,75 | 6 | 32 | 2 | 5 | 5 | 11 | 1 | 13 |
| 17 | 7,5 | 6 | 29 | 0 | - | 2 | 15 | 4 | 11 |
| 19 | 15 | 8 | 9 | 0 | - | 0 | - | 8 | 13 |
| 21 | 7,5 | 7 | 24 | 3 | 6 | 6 | 10 | 2 | 10 |
| 21 | 15 | 7 | 17 | 1 | 15 | 0 | - | 6 | 13 |

n: Anzahl Tiere, x: Mittelwert der Beobachtungen

Die Verbindungen der Formel I können bei der Fertilitätskontrolle als Interzeptiva angewandt werden. Die Verbindungen der Formel I können enteral oder parenteral in Form pharmazeutischer Präparate verabreicht werden. Die Dosierung der Wirkstoffe kann in Abhängigkeit von ihrer Wirkungsstärke etwa 0,2-4 mg/kg bei oraler Applikation betragen. Die pharmazeutischen Präparate können in fester Form, z.B. als Tabletten, Dragées, Kapseln oder Suppositorien oder in flüssiger Form, z.B. in Form von Lösungen vorliegen und eine Verbindung der Formel I zusammen mit einem in solchen Präparaten an sich üblichen inerten anorganischen oder organischen Trägermaterial, wie Wasser, Gelatine, Milchzucker, Stärke, Magnesiumsulfat, Talkum, Polyalkylenglykolen oder Alkoholen enthalten.

Die folgenden Beispiele erläutern die Erfindung weiter.

Beispiel 1

Zu einer Lösung von 1,0 g 17aβ-Hydroxy-4,4-dimethyl-D-homo-androsta-5,16-dien-3-on in 60 ml Benzol gibt man unter Rühren 0,92 g Natriummethylat und anschliessend innert 5 Minuten 2,6 ml Ameisensäureäthylester. Die Mischung wird 4 Stunden bei Raumtemperatur gerührt, dann auf Eiswasser gegossen und mit konz. Salzsäure angesäuert. Man extrahiert dreimal mit Aether, wäscht mit Wasser, trocknet mit $Na_2SO_4$ und dampft das Lösungsmittel im Vakuum ab. Man erhält 1,1 g farblose Kristalle, die mit 250 mg Hydroxylamin-hydrochlorid, 250 mg Natriumacetat und 30 ml Essigsäure 1 Stunde unter Rühren auf 80° erwärmt werden. Das Reaktionsgemisch wird auf Eiswasser gegossen und mit Benzol extrahiert. Den organischen Extrakt wäscht man mit $NaHCO_3$-Lösung und Wasser, trocknet mit $Na_2SO_4$ und dampft das Lösungsmittel im Vakuum ein. Die rohen Kristalle werden aus Aceton-Hexan umkristallisiert und man erhält 850 mg reines 4,4-Dimethyl-D-homo-androsta-2,5,16-trieno-[2,3-d]isoxazol-17aβ-ol vom Schmelzpunkt 200-201°C. $[\alpha]_D^{20}$ = -99° (c = 1,0 in Dioxan). $\varepsilon_{228}$ = 5710.

Das Ausgangsmaterial wird wie folgt hergestellt:

Eine Lösung von 12,0 g 17aβ-Hydroxy-D-homo-androsta-4,16-dien-3-on in 350 ml tert. Butanol wird unter Argon mit 13,4 g Kalium-tert.-butylat versetzt. Anschliessend werden 17 ml Methyljodid zugetropft und die Mischung 1 Stunde unter Rückfluss erhitzt. Zur Aufarbeitung wird auf Eiswasser gegossen, mit Essigester extrahiert, der organische Extrakt mit Wasser gewaschen, $Na_2SO_4$ getrocknet und im Vakuum eingedampft. Der Rückstand wird auf Silicagel chromatographiert. Mit Methylenchlorid-Aceton 98:2 eluiert man 8,9 g reines 17aβ-Hydroxy-4,4-dimethyl-D-homo-androsta-5,16-dien-3-on, Smp. 217-219°C (aus Aceton-Hexan).

## Beispiel 2

Gemäss Beispiel 1 wurde aus 17aβ-Hydroxy-4,4,17a-trimethyl-D-homo-androsta-5,16-dien-3-on die Verbindung 4,4,17a-Trimethyl-D-homo-androsta-2,5,16-trieno-[2,3-d]-isoxazol-17aβ-ol vom Schmelzpunkt 190-191°C (Aceton-Hexan) hergestellt. $[\alpha]_D^{20}$ = -150° (c = 1,0 in Dioxan). $\varepsilon_{229}$ = 5770.

Das Ausgangsmaterial wurde analog Beispiel 1 aus 17aβ-Hydroxy-17a-methyl-D-homo-androsta-4,16-dien-3-on erhalten. Smp. 232-235°C.

## Beispiel 3

Gemäss Beispiel 1 wurde aus 17aβ-Hydroxy-4,4,17a-trimethyl-D-homo-androst-5-en-3-on die Verbindung 4,4,17a-Trimethyl-D-homo-androsta-2,5-dieno-[2,3-d]isoxazol-17aβ-ol vom Schmelzpunkt 210-212°C (aus Aceton-Hexan) hergestellt. $[\alpha]_D^{20}$ = -70° (c = 1,0 in Dioxan). $\varepsilon_{228}$ = 5820.

Das Ausgangsmaterial wurde analog Beispiel 1 aus 17aβ-Hydroxy-17a-methyl-D-homo-androst-4-en-3-on hergestellt. Smp. 212-214°C.

## Beispiel 4

Während einer Stunde wird trockenes Acetylen durch 50 ml auf -70°C abgekühltes Tetrahydrofuran geleitet. Dann gibt man 15 ml 2N Butyllithium-Lösung (in Hexan) zu und tropft anschliessend unter Rühren eine Lösung von 1,75 g 4,4-Dimethyl-D-homo-androsta-2,5,16-trieno-[2,3-d]isoxazol-17a-on in 70 ml Tetrahydrofuran zu. Die Mischung wird 5 Stunden bei -50 bis -40°C gerührt, dann langsam mit 6 ml gesättigter $NH_4Cl$-Lösung versetzt. Nach Erwärmenlassen auf Raumtemperatur wird die Lösung mit $Na_2SO_4$ getrocknet, filtriert und das Filtrat im Vakuum eingedampft. Der Rückstand wird aus Aceton-Hexan umkristallisiert. Man erhält reines 4,4-Dimethyl-D-homopregna-2,5,16-trien-20-yno[2,3-d]-

isoxazol-17aβ-ol, Smp. 253-256°C. $[\alpha]_D^{20}$ = -277° (c = 1,0 in Dioxan).

Das Ausgangsmaterial wurde wie folgt hergestellt:

10,5 g 4,4-Dimethyl-D-homo-androsta-2,5,16-trieno-[2,3-d]isoxazol-17aβ-ol (Beispiel 1), gelöst in 500 ml Aceton, werden bei 0°C innert 5 Minuten mit 8,8 ml $CrO_3$-Lösung (Jones-Reagens) versetzt. Nach 10 Minuten gibt man 10 ml Propanol zu, giesst auf Eiswasser und extrahiert mit Methylenchlorid. Der organische Extrakt wird mit Wasser gewaschen, $Na_2SO_4$ getrocknet und im Vakuum eingedampft. Das Rohprodukt wird aus Aceton-Hexan umkristallisiert. Man erhält 9,1 g reines 4,4-Dimethyl-D-homo-androsta-2,5,16-trieno-[2,3-d]isoxazol-17a-on, Smp. 190-192°C.

## Beispiel 5

3,5 g 2-Hydroxymethylen-17aβ-hydroxy-4,4-dimethyl-D-homo-androsta-5,16-dien-3-on (siehe Beispiel 1) und 1,0 g Hydrazin-hydrat werden in 50 ml Aethanol 1 Stunde unter Rückfluss erhitzt. Die Reaktionsmischung wurde im Vakuum zur Trockene verdampft. DerRückstand wurde aus Aether umkristallisiert. Man erhielt 2,4 g reines 4,4-Dimethyl-2'H-D-homoandrosta-2,5,16-trieno-[3,2-c]pyrazol-17aβ-ol, Smp. 169-170°C. $[\alpha]_D^{20}$ = -80° (c = 0,5 in Dioxan). $\varepsilon_{222}$ = 4950.

## Beispiel 6

Zu einer auf 0°C abgekühlten Lösung von 1,55 g 4,4-Dimethyl-2'H-D-homo-androsta-2,5,16-trieno-[3,2-c]pyrazol-17a-on in 40 ml abs. Tetrahydrofuran und 40 ml abs. Aether gibt man innert 10 Minuten 3,85 ml einer ca. 2-molaren $LiCH_3$-Lösung. Nach 2 Stunden Rühren bei 0°C wird die Reaktionsmischung mit 5 ml Natriumsulfatlösung versetzt, dann auf Eiswasser gegossen und mit Methylenchlorid extrahiert. Der organische Extrakt wird mit Wasser gewaschen, $Na_2SO_4$ getrocknet und im Vakuum eingedampft. Das Rohprodukt wird

auf Silicagel chromatographiert. Mit Methylenchlorid-Aceton 9:1 eluiert man 700 mg reines 4,4,17a-Trimethyl-2'H-D-homo-androsta-2,5,16-trieno[3,2-c]pyrazol-17aβ-ol vom Schmelzpunkt 268-270°C. $[\alpha]_D^{20}$ = -133° (c = 0,5 in Dioxan). $\varepsilon_{223}$ = 5060.

Das Ausgangsmaterial wird wie folgt hergestellt:

Zu einer auf -20°C abgekühlten Lösung von 4,8 g 4,4-Dimethyl-2'H-D-homo-androsta-2,5,16-trieno[3,2-c]pyrazol-17aβ-ol in 190 ml Aceton gibt man innert 10 Minuten 4,1 ml Jones-Reagens. Uebliche Aufarbeitung ergibt nach Chromatographie an Silicagel reines 4,4-Dimethyl-2'H-D-homoandrosta-2,5,16-trieno[3,2-c]pyrazol-17a-on, Smp. 287-288°.

## Beispiel 7

Gemäss Beispiel 6 wird unter Verwendung von Butyllithium an Stelle von Methyllithium aus 4,4-Dimethyl-2'H-D-homoandrosta-2,5,16-trieno[3,2-c]pyrazol-17a-on reines 17a-Butyl-4,4-dimethyl-2'H-D-homoandrosta-2,5,16-trieno-[3,2-c]pyrazol-17aβ-ol vom Schmelzpunkt 196-198°C (Aether) erhalten. $[\alpha]_D^{20}$ = -151° (c = 0,3 in Dioxan). $\varepsilon_{223}$ = 5230.

## Beispiel 8

Gemäss Beispiel 6 wird unter Verwendung von Butyllithium an Stelle von Methyllithium aus 4,4-Dimethyl-D-homo-androsta-2,5,16-trieno[2,3-d]isoxazol-17a-on reines 17a-Butyl-4,4-dimethyl-D-homo-androsta-2,5,16-trieno[2,3-d]-isoxazol-17aβ-ol in amorpher Form erhalten. $[\alpha]_D^{20}$ = -574° (c = 0,066 in Dioxan). $\varepsilon_{226}$ = 7000.

## Beispiel 9

Gemäss Beispiel 4 wird aus 4,4-Dimethyl-2'H-D-homo-androsta-2,5,16-trieno[3,2-c]pyrazol-17a-on reines 4,4-Dimethyl-2'H-D-homo-17aα-pregna-2,5,16-trien-20-yno[3,2-c]-pyrazol-17a-ol vom Schmelzpunkt 269-271°C (Aceton) erhalten. $[\alpha]_D^{20}$ = -257° (c = 1,0 in Dioxan). $\varepsilon_{222}$ = 5770.

Beispiel 10

Gemäss Beispiel 1 wurde aus 4,5-Dihydro-4',4'-dimethylspiro[furan-2(3H),17'a(betal)-D-homoandrosta-[5,16]-dien]-3-on reines 4,5-Dihydro-4',4'-dimethylspiro[furan-2(3H),17'a(beta 1)-D-homoandrosta[2,5,16]trieno[2,3-d]isoxazol, Smp. 196-197°C (Aceton-Hexan) erhalten. $[\alpha]_D^{20}$ = +165° (c = 0,9 in Dioxan). $\varepsilon_{228}$ = 5890.

Das Ausgangsmaterial wird gemäss Beispiel 1 durch Methylierung von 4,5-Dihydro-spiro[furan-2(3H),17'a(beta 1)-D-homoandrosta[4,16]dien]-3'-on hergestellt. Smp. 140-142°C. $[\alpha]_D^{20}$ = -131° (c = 0,5 in Dioxan).

Beispiel 11

Eine Mischung von 1,0 g 17β-Hydroxy-4,4,17-trimethyl-D-homo-androsta-5,16-dien-2,3-dion, 1,0 g Hydroxylaminhydrochlorid und 50 ml Pyridin wurde 6 Stunden auf 100°C erwärmt. Die Reaktionsmischung wurde im Vakuum eingedampft, der Rückstand in Methylenchlorid gelöst und die Methylenchlorid-Lösung nacheinander mit eiskalter verdünnter Salzsäure, Wasser, gesättigter $NaHCO_3$-Lösung und Wasser gewaschen und dann mit $Na_2SO_4$ getrocknet. Nach Eindampfen des Lösungsmittels erhielt man 1,0 g kristallines Dioxan, das mit 50 ml Aethylenglykol und 2,0 g Kaliumhydroxid 90 Minuten auf 160°C erhitzt wurde. Das Reaktionsgemisch wurde auf Eiswasser gegossen, mit Salzsäure angesäuert und mit Methylenchlorid extrahiert. Der organische Extrakt wurde mit Wasser gewaschen, mit $Na_2SO_4$ getrocknet und eingedampft. Durch Umkristallisation aus Aceton-Hexan wurden 0,8 g reines 4,4,17a-Trimethyl-D-homo-androsta-5,16-dieno-[2,3-d]-furazan-17β-ol vom Schmelzpunkt 178-180°C erhalten. $[\alpha]_D^{25}$ = -137° (c = 0,4 in Dioxan). $\varepsilon_{215}$ = 5640.

Das Ausgangsmaterial wurde wie folgt hergestellt:

10 g 17aβ-Hydroxy-4,4,17a-trimethyl-D-homo-androsta-5,16-dien-3-on und 15 g Kalium-tert.butylat wurden in 600 ml tert. Butanol gelöst und auf 70°C erwärmt. Unter Rühren wurde während 2 1/2 Stunden Luft durch die Lösung geleitet. Zur Aufarbeitung goss man auf Eiswasser, säuerte mit Essigsäure an und extrahierte mit Methylenchlorid. Der organische Extrakt wurde mit Wasser gewaschen, $Na_2SO_4$ getrocknet und im Vakuum eingedampft. Der Rückstand wurde auf Silicagel mit Hexan-Aceton 9:1 chromatographiert und lieferte 6,5 g reines 17β-Hydroxy-4,4,17-trimethyl-D-homo-androsta-5,16-dien-2,3-dion, Smp. 155-157°C.

## Beispiel 12

Zu einer auf -15°C abgekühlten Lösung von 150 mg 4,4-Dimethyl-2'H-D-homo-androsta-2,5,16-trieno][3,2-c]-pyrazol-17aβ-ol und 50 mg 4-Dimethylaminopyridin in 1,5 ml Triäthylamin tropft man 1,5 ml Essigsäureanhydrid. Man rührt 90 Minuten bei -15°C, giesst dann auf Eiswasser und extrahiert mit Methylenchlorid. Die organische Phase wird mit Wasser gewaschen, $Na_2SO_4$ getrocknet und im Vakuum eingedampft. Das Rohprodukt wird auf 10 g Silicagel chromatographiert. Mit Methylenchlorid-Aceton 99:1 werden 160 mg reines amorphes, 1'-Acetyl-4,4-dimethyl-1'H-D-homoandrosta-5,16-dieno-[3,2-c]pyrazol-17aβ-ylacetat eluiert. $[\alpha]_D^{20}$ = -63° (c = 0,4 in Dioxan). $\varepsilon_{253}$ = 19320.

## Beispiel 13

Gemäss Beispiel 12 wird aus 4,4-Dimethyl-2'H-D-homo-17aα-pregna-2,5,16-trien-20-yno-[3,2-c]pyrazol-17a-ol (Beispiel 9) reines 1'-Acetyl-4,4-dimethyl-1'H-D-homo-17aα-pregna-5,16-dien-20-yno[3,2-c]pyrazol-17a-ol erhalten. Smp. 238-240°C. $[\alpha]_D^{20}$ = -240° (c = 0,3 in Dioxan). $\varepsilon_{257}$ = 19100.

Beispiel 14

Eine Mischung von 900 mg 17aβ-Hydroxy-4,4,17a-trimethyl-D-homo-androsta-2,5-dieno-[2,3-d]isoxazol-17aβ-ol, 100 ml abs. Aether, 1 ml Methanol und 210 mg Natriumäthylat wird 5 Stunden bei Raumtemperatur gerührt. Zur Aufarbeitung wird auf Eiswasser gegossen, mit verdünnter Salzsäure angesäuert und mit Aether extrahiert. Der Aether-Extrakt wird mit Wasser gewaschen, $Na_2SO_4$ getrocknet und im Vakuum eingedampft. Das Rohprodukt kristallisiert man aus Aether um und man erhält 650 mg reines 17aβ-Hydroxy-4,4,17a-trimethyl-3-oxo-D-homo-androst-5-en-2α-carbonitril vom Schmelzpunkt 196-197°C. $[\alpha]_D^{20}$ = -34° (c = 1,0 in Dioxan). $\varepsilon_{236}$ = 7800.

Beispiel 15

Gemäss Beispiel 14 wird aus 4,4-Dimethyl-D-homo-17aα-pregna-2,5,16-trien-20-yno[3,2-d]isoxazol-17a-ol reines 17a-Hydroxy-4,4-dimethyl-3-oxo-D-homo-17aα-pregna-5,16-dien-20-yn-2α-carbonitril erhalten. Smp. 241-244°C (Aceton-Hexan). $[\alpha]_D^{20}$ = -25° (c = 1,0 in Dioxan). $\varepsilon_{237}$ = 7600.

Beispiel 16

Gemäss Beispiel 14 wird aus 4,5-Dihydro-4',4'-dimethyl-spiro[furan-2(3H),17'a-(beta 1)-D-homoandrosta[2,5,16]trieno-[2,3-d]isoxazol reines 4,5-Dihydro-4',4'-dimethyl-3'-oxo-spiro[furan-2(3H),17'a(beta 1)-D-homoandrosta[5,16]dien]-2α-carbonitril vom Schmelzpunkt 158-160°C erhalten. $[\alpha]_D^{20}$ = -139° (c = 0,3 in Dioxan). $\varepsilon_{238}$ = 7400.

Beispiel 17

Eine Lösung von 65 g 2-Hydroxymethylen-17aβ-hydroxy-4,4,17a-trimethyl-D-homo-androsta-5,16-dien-3-on und 84 g O,N-Bis(trifluoracetyl)-hydroxylamin in 1800 ml Benzol und 180 ml Pyridin werden 4 Stunden unter Argon zum Sieden erhitzt. Zur Aufarbeitung wird auf Eiswasser gegossen und mit Benzol extrahiert. Die Benzollösung wäscht man mit Wasser, trocknet mit $Na_2SO_4$ und dampft das Lösungsmittel im Vakuum ein. Der Rückstand wird auf 2 kg Silicagel chromatographiert. Mit Hexan-Aceton 9:1 eluiert man vorerst 5,9 g 4,4,17a-Trimethyl-D-homo-androsta-2,5,16-trieno-[2,3-d]-isoxazol-17aβ-ol (Beispiel 2) und anschliessend 34 g reines 17aβ-Hydroxy-4,4,17a-trimethyl-3-oxo-D-homo-androsta-5,16-dien-2α-carbonitril vom Schmelzpunkt 178-180°C (aus Aether). $[\alpha]_D^{20} = -121°$ (c = 0,3 in Dioxan). $\varepsilon_{237} = 6950$.

Beispiel 18

Eine Lösung von 200 mg 17aβ-Hydroxy-4,4,17a-trimethyl-3-oxo-D-homo-androsta-5,16-dien-2α-carbonitril in 5 ml Pyridin und 5 ml Essigsäureanhydrid wird 2 Stunden bei Raumtemperatur gehalten. Zur Aufarbeitung wird auf Eiswasser gegossen und mit Methylenchlorid extrahiert. Die Extraktionslösung wird mit Wasser gewaschen, $Na_2SO_4$ getrocknet und im Vakuum eingedampft. DerRückstand wird aus Aceton-Hexan umkristallisiert. Man erhält 170 mg reines 3-Acetoxy-17aβ-hydroxy-4,4,17a-trimethyl-D-homo-androsta-2,5,16-trien-2-carbonitril vom Schmelzpunkt 184-186°C. $[\alpha]_D^{20} = -141°$ (c = 0,5 in Dioxan). $\varepsilon_{217} = 9700$.

Beispiel 19

Zu einer auf -30°C abgekühlten Lösung von 1,75 g 4,4-Dimethyl-D-homo-androsta-2,5,16-trieno-[2,3-d]isoxazol-17a-on in 50 ml abs. Tetrahydrofuran gibt man 400 mg Lithium in kleinen Stücken. Danach tropft man bei -30°C innerhalb von 40 Minuten 4,0 g 3-Brompropionaldehyddimethylacetal zu. Dann wird noch 1 Stunde bei -30°C und 1 Stunde bei 0°C gerührt. Zur Aufarbeitung wird überschüssiges Lithium abfiltriert und das Filtrat auf Eiswasser gegossen und mit Essigester extrahiert. Der organische Extrakt wird mit Wasser gewaschen, mit $Na_2SO_4$ getrocknet und im Vakuum eingedampft. Man erhält 2,6 g gelbes,

amorphes Produkt, das in 100 ml Essigsäure und 20 ml Wasser gelöst, 2 Stunden bei Raumtemperatur gerührt wird. Dann giesst man die Mischung auf Eiswasser und extrahiert mit Essigester. Der Extrakt wird mit $Na_2CO_3$-Lösung und Wasser gewaschen, mit $Na_2SO_4$ getrocknet und im Vakuum eingedampft. Man erhält 2,20 g amorphes Lactol, das in 50 ml Methylenchlorid und 50 ml Aceton gelöst bei 15°C mit 1,90 ml Jones-Reagens versetzt wird. Die Mischung wird 40 Minuten bei 15°C gerührt, dann mit 2 ml 2-Propanol versetzt und anschliessend auf Eis-Kochsalzlösung gegossen. Man extrahiert mit Methylenchlorid, wäscht die Extraktlösung mit Wasser, trocknet mit $Na_2SO_4$ und dampft im Vakuum ein. Man erhält 2,1 g Rohprodukt, das auf 100 g Silicagel chromatographiert wird. Mit Methylenchlorid-Aceton 99:1 kann man 940 mg reines 2α-Cyano-4,4-dimethyl-3-oxo-D-homo-17aα-pregna-5,16-dien-21,17a-carbolacton eluieren. Smp. 203-205°C. $[\alpha]_D^{20}$ = -114° (c = 0,5 in Dioxan). $\varepsilon_{238}$ = 6950.

Beispiel 20

Zu einer Lösung von 1,0 g 17aβ-Hydroxy-4,4,17a-trimethyl-D-homo-androsta-5,16-dien-3-on und 330 mg Kalium-tert,butylat in 60 ml tert.Butanol gibt man 0,48 ml tert.-Butylnitrit. Die Mischung wird bei 60°C unter Argon 5 Stunden gerührt. Zur Aufarbeitung wird im Vakuum eingeengt, der Rückstand in Methylenchlorid aufgenommen, die Methylenchlorid-Lösung mit Wasser gewaschen, mit $Na_2SO_4$ getrocknet und im Vakuum eingedampft. Man erhält 1,0 g Rohprodukt, das auf Silicagel chromatographiert (Hexan-Aceton 7:1) 850 mg reines 17aβ-Hydroxy-4,4,17a-trimethyl-D-homoandrosta-5,16-dien-2,3-dion-2-oxim vom Schmelzpunkt 248°C liefert. $[\alpha]_D^{20}$ = -6° (c = 0,2 in Dioxan). $\varepsilon_{237}$ = 8100.

Beispiel 21

Eine Mischung von 370 mg 17aβ-Hydroxy-4,4,17a-trimethyl-D-homo-androsta-5,16-dien-3-on, 83 mg Hydroxylaminhydrochlorid, 98 mg Natriumacetat und 10 ml DMSO wurde

96 Stunden unter Argon bei Raumtemperatur gerührt. Zur Aufarbeitung wurde auf Eiswasser gegossen und mit Benzol extrahiert. Der Benzolextrakt wird mit Wasser gewaschen, mit $Na_2SO_4$ getrocknet und im Vakuum eingedampft. Der Rückstand wird auf 20 g Silicagel chromatographiert. Mit Hexan-Aceton 8:1 eluiert man 157 mg reines 17aβ-Hydroxy-2-[(hydroxyamino)methylen]-4,4,17a-trimethyl-D-homoandrosta-5,16-dien-3-on, Smp. 156-160°C. $[\alpha]_D^{25}$ = -10° (c = 0,5 in Dioxan). $\varepsilon_{285}$ = 4500.

## Beispiel 22

Zu einer auf 0°C abgekühlten Lösung von 3,17aβ-Diacetoxy-2-cyano-D-homo-androsta-2,4,16-trien in 100 ml Aether gibt man 740 mg m-Chlorperbenzoesäure. Die Mischung wird 22 Stunden bei Raumtemperatur gerührt, dann nochmals mit 470 mg m-Chlorperbenzoesäure versetzt. Nach weiteren 8 Stunden giesst man auf Eiswasser-$NaHCO_3$-Lösung und extrahiert mit Aether. Der Aetherextrakt wird mit Wasser gewaschen, mit $Na_2SO_4$ getrocknet und im Vakuum eingedampft. Das Rohprodukt wird aus Aceton-Hexan umkristallisiert. Man erhält 1,0 g reines 3,17aβ-Diacetoxy-4α,5α-epoxy-D-homo-5α-androsta-2,16-dien-2-carbonitril, Smp. 218-220°C. $[\alpha]_D^{20}$ = +40° (c = 1,0 in Dioxan). $\varepsilon_{240}$ = 9020.

Das Ausgangsmaterial wird wie folgt hergestellt:

17aβ-Hydroxy-D-homo-androsta-4,16-dien-3-on wird mit Ameisensäureäthylester und Natriumhydrid in die 2-Hydroxymethylen-Verbindung umgesetzt, welche mit Hydroxylaminhydrochlorid in Aethanol D-Homoandrosta-2,4,16-trieno-[2,3-d]isoxazol-17aβ-ol vom Schmelzpunkt 194-196°C liefert. Diese Verbindung wird mit Natriummethylat in Aether und Methanol zum 17aβ-Hydroxy-3-keto-D-homoandrosta-4,16-dien-2α-carbonitril vom Schmelzpunkt 144-147°C gespalten. Acetylierung mit Acetanhydrid in Pyridin liefert das gewünschte 3,17aβ-Diacetoxy-2-cyano-D-homo-androsta-2,4,16-trien.

## Beispiel 23

Eine Mischung von 500 mg 3,17aβ-Diacetoxy-4α,5α-epoxy-D-homo-5α-androsta-2,16-dien-2-carbonitril, 500 mg Kaliumcarbonat, 500 ml Methanol und 5 ml Wasser wird 1 Stunde unter Argon bei Raumtemperatur gerührt. Zur Aufarbeitung wird die Reaktionsmischung auf Eiswasser gegossen und mit Methylenchlorid extrahiert. Der organische Extrakt wird mit Wasser gewaschen, $Na_2SO_4$ getrocknet und im Vakuum eingedampft. Der Rückstand wird aus Aceton-Hexan umkristallisiert. Man erhält 265 mg reines 17aβ-Acetoxy-4α,5-epoxy-3-oxo-D-homo-5α-androst-16-en-2α-carbonitril vom Schmelzpunkt 191-193°C. $[\alpha]_D^{20}$ = +53° (c = 0,5 in Dioxan). $\varepsilon_{254}$ = 6980.

## Beispiel 24

Gemäss Beispiel 22 wurde aus 3-Acetoxy-17aβ-hydroxy-4,17a-dimethyl-D-homo-androsta-2,4,16-trien-2-carbonitril reines 3-Acetoxy-4α,5-epoxy-17aβ-hydroxy-4,17a-dimethyl-D-homo-5α-androsta-2,16-dien-2-carbonitril erhalten. Smp. 192-193°C (Aether-Hexan). $[\alpha]_D^{20}$ = -3° (c = 0,3 in Dioxan). $\varepsilon_{240}$ = 8460.

Das Ausgangsmaterial wird wie folgt hergestellt:

17aβ-Hydroxy-17aα-methyl-D-homo-androsta-4,16-dien-3-on wird mit Methyljodid/Kalium-tert.butylat zu 17aβ-Hydroxy-4,17aα-methyl-D-homo-androsta-4,16-dien-3-on vom Schmelzpunkt 152-153°C methyliert. Umsetzung mit Ameisensäureäthylester-Natriummethylat zur 2-Hydroxymethylen-Verbindung und anschliessende Reaktion mit O,N-Bis(trifluoracetyl)hydroxylamin gemäss Beispiel 17 liefert 17aβ-Hydroxy-4,17a-dimethyl-D-homo-androsta-4,16-dien-2α-carbonitril als amorphe Substanz. Umsetzung mit Acetanhydrid-Pyridin liefert schliesslich reines 3-Acetoxy-17aβ-hydroxy-4,17a-dimethyl-D-homo-androsta-2,4,16-trien-2-carbonitril vom Schmelzpunkt 210-212°C.

## Beispiel 25

Gemäss Beispiel 23 wurde aus 3-Acetoxy-4α,5-epoxy-17aβ-hydroxy-4,17a-dimethyl-D-homo-5α-androsta-2,16-dien-2-carbonitril reines 4α,5-Epoxy-17aβ-hydroxy-4,17a-dimethyl-3-oxo-D-homo-5α-androst-16-en-2α-carbonitril erhalten. Smp. 190-192°C (aus Aceton-Hexan). $[\alpha]_D^{20}$ = -88° (c = 0,3 in Dioxan). $\varepsilon_{251}$ = 4880.

## Beispiel A

Eine Tablette zur oralen Verabreichung kann die folgende Zusammensetzung aufweisen:

| | |
|---|---|
| Wirkstoff, z.B. 17aβ-Hydroxy-4,4,17a-trimethyl-3-oxo-D-homoandrosta-5,16-dien-2β-carbonitril | 20 mg |
| Milchzucker | 120 mg |
| Maisstärke | 80 mg |
| Talkum | 4 mg |
| Magnesiumstearat | 1 mg |

## Patentansprüche

1. D-Homosteroide der Formel

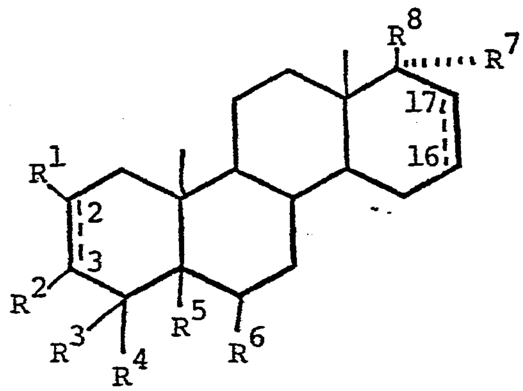

I

worin $R^1$ einen Rest -CN, =NOH oder =CH-NHOH; $R^2$ Oxo oder, falls $R^1$ ein Rest -CN ist, Oxo oder einen Rest -OAc; oder $R^1$ und $R^2$ zusammen mit den C-Atomen 2 und 3 des Steroidgerüsts einen [2,3-d]-annelierten Isoxazolring, einen [3,2-c]-annelierten Pyrazolring, einen [3,2-c]-annelierten N-acylierten Pyrazolring oder einen [2,3-c]-annelierten Furazanring; Ac einen Acylrest; $R^3$ und $R^4$ Methyl und $R^5$ und $R^6$ gemeinsam eine zusätzliche Bindung zwischen den C-Atomen 5 und 6 des Steroidgerüsts; oder $R^4$ und $R^5$ gemeinsam -O-, $R^3$ Wasserstoff oder Methyl, und $R^6$ Wasserstoff; $R^7$ Wasserstoff, nieder-Alkyl oder Aethinyl; $R^8$ Hydroxy oder Acyloxy; oder $R^7$ und $R^8$ gemeinsam einen Spiroätherrest der Formel

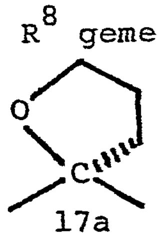

oder einen Spirolactonrest der Formel

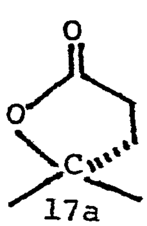

darstellen, die punktierte 16,17-Bindung eine fakultative zusätzliche C-C-Bindung ist;

und die punktierte 2,3-Bindung eine zusätzliche C-C-Bindung ist, wenn $R^2$ und $R^3$ zusammen mit den C-Atomen 2 und 3 den Isoxazol- oder Pyrazolring bilden oder wenn $R^2$ ein Rest -OAc ist.

2. 16,17-ungesättigte D-Homosteroide der Formel I von Anspruch 1.

3. D-Homosteroide der Formel I gemäss Anspruch 1, in denen $R^1$ Cyano ist.

4. 17aβ-Hydroxy-4,4,17a-trimethyl-3-oxo-D-homo-androsta-5,16-dien-2β-carbonitril.

5. 17aβ-Hydroxy-4,4,17a-trimethyl-3-oxo-D-homo-androst-5-en-2α-carbonitril, 17a-Hydroxy-4,4-dimethyl-3-oxo-D-homo-17aα-pregna-5,16-dien-20-yn-2α-carbonitril, 4,5-Dihydro-4',4'-dimethyl-3'-oxo-spiro[furan-2(3H),17'a(beta 1)-D-homoandrosta[5,16]-dien]-2α-carbonitril, 3-Acetoxy-17aβ-hydroxy-4,4,17a-trimethyl-D-homo-androsta-2,5,16-trien-2-carbonitril, 2α-Cyano-4,4-dimethyl-3-oxo-D-homo-17aα-pregna-5,16-dien-21,17a-carbolacton, 3,17aβ-Diacetoxy-4α,5α-epoxy-D-homo-5α-androsta-2,16-dien-2-carbonitril, 17aβ-Acetoxy-4α,5-epoxy-3-oxo-D-homo-5α-androst-16-en-2α-carbonitril, 3-Acetoxy-4α,5-epoxy-17aβ-hydroxy-4,17a-dimethyl-D-homo-5α-androsta-2,16-dien-2-carbonitril und 4α,5-Epoxy-17aβ-hydroxy-4,17a-dimethyl-3-oxo-D-homo-5α-androst-16-en-2α-carbonitril.

6. 4,4-Dimethyl-D-homo-androsta-2,5,16-trieno-[2,3-d]-isoxazol-17aβ-ol, 4,4,17a-Trimethyl-D-homo-androsta-2,5,16-trieno-[2,3-d]isoxazol-17aβ-ol, 4,4,17a-Trimethyl-D-homo-androsta-2,5-dieno-[2,3-d]isoxazol-17aβ-ol, 4,4-Dimethyl-D-homopregna-2,5,16-trien-20-yno[2,3-d]isoxazol-17aβ-ol, 4,4-Dimethyl-2'H-D-homoandrosta-2,5,16-trieno-[3,2-c]-pyrazol-17aβ-ol, 4,4,17a-Trimethyl-2'H-D-homo-androsta-2,5,16-trieno[3,2-c]pyrazol-17aβ-ol, 17a-Butyl-4,4-dimethyl-2'H-D-homoandrosta-2,5,16-trieno[3,2-c]pyrazol-17aβ-ol,

17a-Butyl-4,4-dimethyl-D-homo-androsta-2,5,16-trieno[2,3-d]-isoxazol-17aβ-ol, 4,4-Dimethyl-2'H-D-homo-17aα-pregna-2,5,16-trien-20-yno[3,2-c]pyrazol-17a-ol, 4,5-Dihydro-4',4'-dimethylspiro[furan-2(3H),17'a(beta 1)-D-homoandrosta-[2,5,16]trieno[2,3-d]isoxazol, 4,4,17a-Trimethyl-D-homo-androsta-5,16-dieno-[2,3-d]furazan-17β-ol, 4,4-Dimethyl-2'H-D-homo-androsta-2,5,16-trieno][3,2-c]pyrazol-17aβ-ol und 1'-Acetyl-4,4-dimethyl-1'H-D-homo-17aα-pregna-5,16-dien-20-yno[3,2-c]pyrazol-17a-ol.

7. 17aβ-Hydroxy-4,4,17a-trimethyl-D-homoandrosta-5,16-dien-2,3-dion-2-oxim und 17aβ-Hydroxy-2-[(hydroxyamino)-methylen]-4,4,17a-trimethyl-D-homoandrosta-5,16-dien-3-on.

8. D-Homosteroide der Formel I gemäss Anspruch 1 zur Anwendung als pharmazeutische Wirkstoffe.

9. D-Homosteroide der Formel I gemäss Anspruch 1 zur Anwendung als Interzeptiva .

10. Pharmazeutische Präparate, enthaltend ein D-Homosteroid der Formel I gemäss Anspruch 1 und einen Trägerstoff.

11. Verfahren zur Herstellung der D-Homosteroide der Formel I von Anspruch 1, dadurch gekennzeichnet, dass man

a) ein D-Homosteroid der Formel

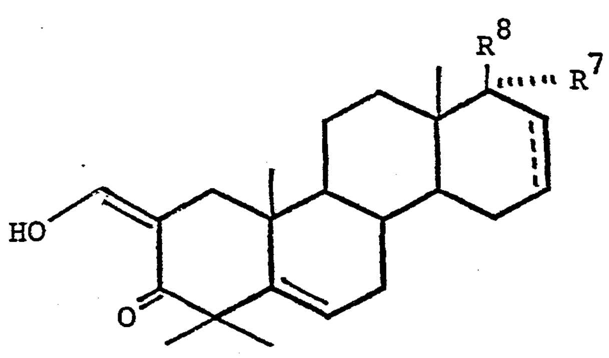

II

 

mit Hydroxylamin oder Hydrazin oder O,N-Bis-(trifluoracetyl)-hydroxylamin umsetzt; oder

b) ein D-Homosteroid der Formel

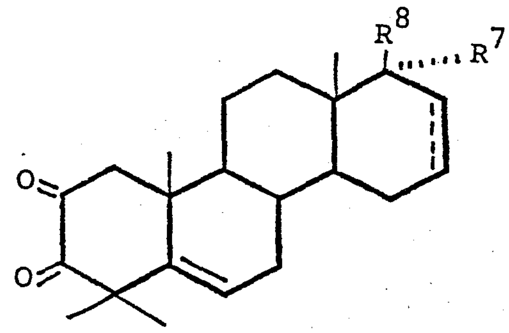

III

mit Hydroxylamin umsetzt; oder

c) ein D-Homosteroid der Formel

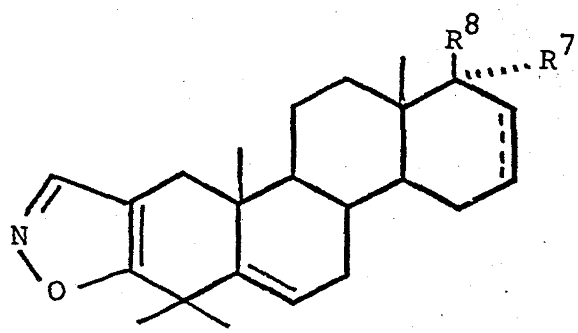

IV

mit einer starken Base behandelt; oder

d) ein D-Homosteroid der Formel

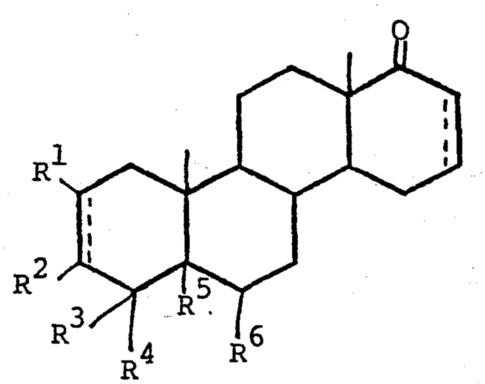

V

mit einer den Rest $R^7$ abgebenden metallorganischen Verbindung umsetzt; oder

e) ein D-Homosteroid der Formel

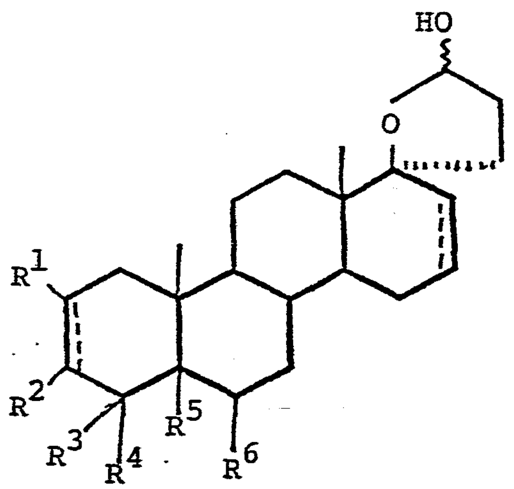

VI

mit einem Oxidationsmittel behandelt; oder

f) ein D-Homosteroid der Formel I, worin $R^1$ Cyano und $R^2$ Oxo oder $R^1$ und $R^2$ zusammen mit den C-Atomen 2 und 3 des Steroidgerüsts einen Pyrazolring darstellen und/oder $R^8$ Hydroxy ist; mit einem Acylierungsmittel behandelt, oder

g) ein D-Homosteroid der Formel

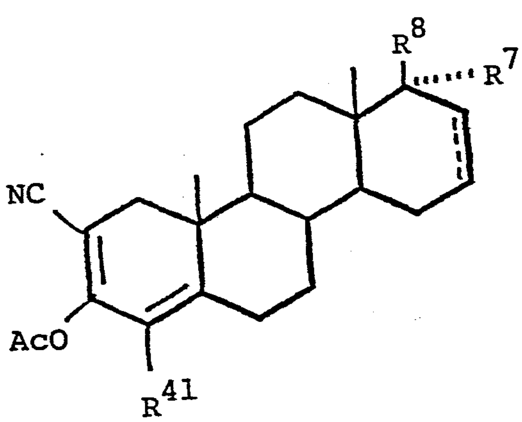

VII

mit einem Oxidationsmittel behandelt und das erhaltene 4,5-Oxid gewünschtenfalls mit einer Base behandelt, wobei in den vorstehenden Formeln II-VII Ac einen Acylrest; $R^{41}$ Wasserstoff oder Methyl darstellen; und $R^1$-$R^8$ die in Anspruch 1 angegebene Bedeutung haben.

***

Patentansprüche für Oesterreich

1. Verfahren zur Herstellung von D-Homosteroiden der Formel

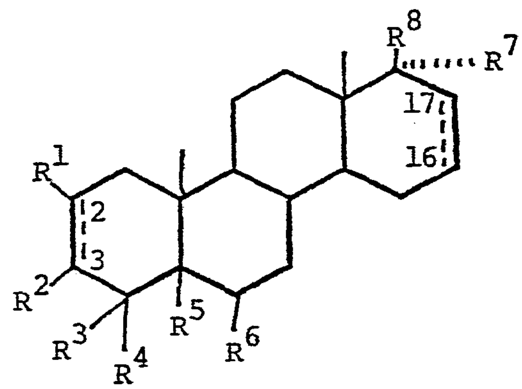

I

worin $R^1$ einen Rest -CN, =NOH oder =CH-NHOH; $R^2$ Oxo oder, falls $R^1$ ein Rest -CN ist, Oxo oder einen Rest -OAc; oder $R^1$ und $R^2$ zusammen mit den C-Atomen 2 und 3 des Steroidgerüsts einen [2,3-d]-annelierten Isoxazolring, einen [3,2-c]-annelierten Pyrazolring, einen [3,2-c]-annelierten N-acylierten Pyrazolring oder einen [2,3-c]-annelierten Furazanring; Ac einen Acylrest; $R^3$ und $R^4$ Methyl und $R^5$ und $R^6$ gemeinsam eine zusätzliche Bindung zwischen den C-Atomen 5 und 6 des Steroidgerüsts; oder $R^4$ und $R^5$ gemeinsam -O-, $R^3$ Wasserstoff oder Methyl, und $R^6$ Wasserstoff; $R^7$ Wasserstoff, nieder-Alkyl oder Aethinyl; $R^8$ Hydroxy oder Acyloxy; oder $R^7$ und $R^8$ gemeinsam einen Spiroätherrest der Formel

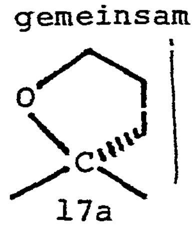

oder einen Spirolactonrest der Formel

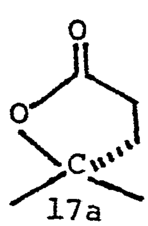

darstellen, die punktierte 16,17-Bindung eine fakultative zusätzliche C-C-Bindung ist; und die punktierte 2,3-Bindung eine zusätzliche C-C-Bindung ist, wenn $R^2$ und $R^3$ zusammen mit den C-Atomen 2 und 3 den Isoxazol- oder Pyrazolring bilden oder wenn $R^2$ ein Rest -OAc ist,

dadurch gekennzeichnet, dass man

a) ein D-Homosteroid der Formel

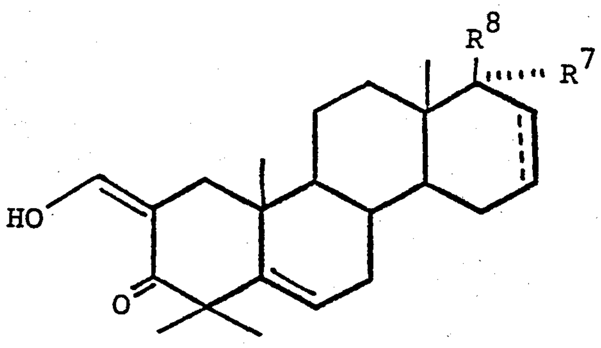

II

mit Hydroxylamin oder Hydrazin oder O,N-Bis-(trifluoracetyl)-hydroxylamin umsetzt; oder

b) ein D-Homosteroid der Formel

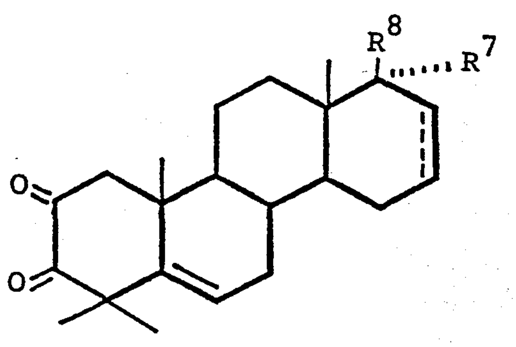

III

mit Hydroxylamin umsetzt; oder

c) ein D-Homosteroid der Formel

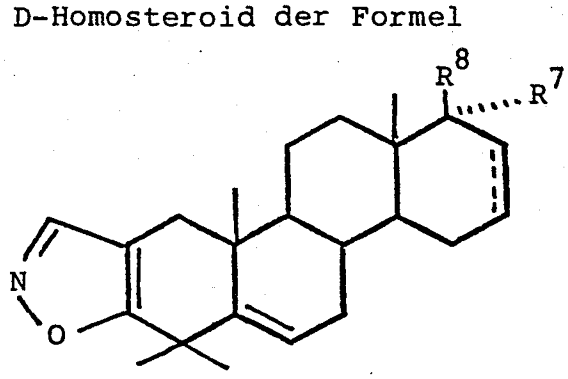

IV

mit einer starken Base behandelt; oder

d) ein D-Homosteroid der Formel

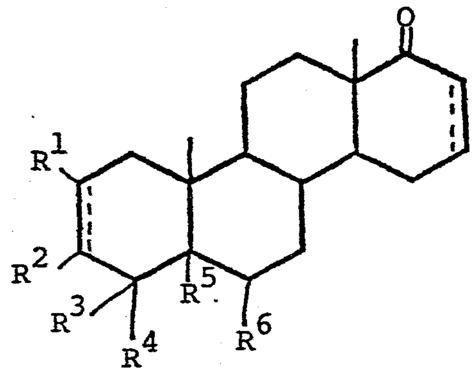

V

mit einer den Rest $R^7$ abgebenden metallorganischen Verbindung umsetzt; oder

e) ein D-Homosteroid der Formel

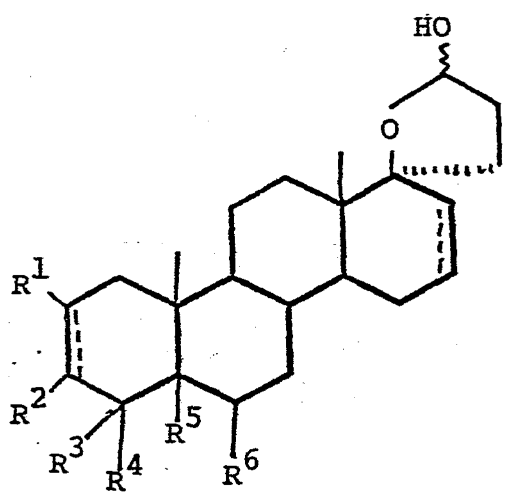

VI

mit einem Oxidationsmittel behandelt; oder

f) ein D-Homosteroid der Formel I, worin $R^1$ Cyano und $R^2$ Oxo oder $R^1$ und $R^2$ zusammen mit den C-Atomen 2 und 3 des Steroidgerüsts einen Pyrazolring darstellen und/oder $R^8$ Hydroxy ist; mit einem Acylierungsmittel behandelt, oder

g) ein D-Homosteroid der Formel

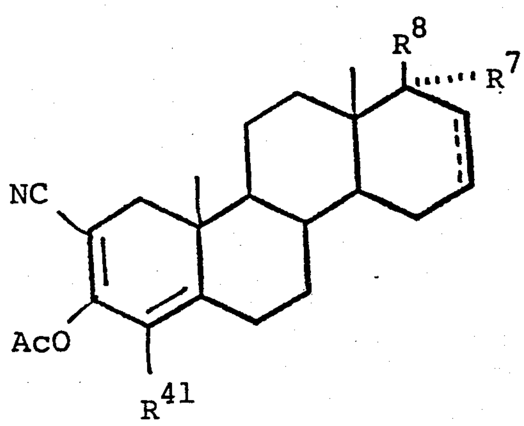

VII

mit einem Oxidationsmittel behandelt und das erhaltene 4,5-Oxid gewünschtenfalls mit einer Base behandelt, wobei in den vorstehenden Formeln II-VII Ac einen Acylrest; $R^{41}$ Wasserstoff oder Methyl darstellen; und $R^1$-$R^8$ die oben angegebene Bedeutung haben.

2. Verfahren nach Anspruch 1, dadurch gekennzeichnet, dass man 16,17-ungesättigte D-Homosteroide der Formel I herstellt.

3. Verfahren nach Anspruch 1, dadurch gekennzeichnet, dass man D-Homosteroide der Formel I herstellt, in denen $R^1$ Cyano ist.

4. Verfahren nach Anspruch 1, dadurch gekennzeichnet, dass man 17aβ-Hydroxy-4,4,17a-trimethyl-3-oxo-D-homo-androsta-5,16-dien-2β-carbonitril herstellt.

5. Verfahren nach Anspruch 1, dadurch gekennzeichnet, dass man 17aβ-Hydroxy-4,4,17a-trimethyl-3-oxo-D-homo-androst-5-en-2α-carbonitril, 17a-Hydroxy-4,4-dimethyl-3-oxo-D-homo-17aα-pregna-5,16-dien-20-yn-2α-carbonitril, 4,5-Dihydro-4',4'-dimethyl-3'-oxo-spiro[furan-2(3H),17'a(beta 1)-D-homoandrosta[5,16]-dien]-2α-carbonitril, 3-Acetoxy-17aβ-hydroxy-4,4,17a-trimethyl-D-homo-androsta-2,5,16-trien-2-carbonitril, 2α-Cyano-4,4-dimethyl-3-oxo-D-homo-17aα-pregna-5,16-dien-21,17a-carbolacton, 3,17aβ-Diacetoxy-4α,5α-epoxy-D-homo-5α-androsta-2,16-dien- 2-carbonitril,

17aβ-Acetoxy-4α,5-epoxy-3-oxo-D-homo-5α-androst-16-en-2α-carbonitril, 3-Acetoxy-4α,5-epoxy-17aβ-hydroxy-4,17a-dimethyl-D-homo-5α-androsta-2,16-dien-2-carbonitril und 4α,5-Epoxy-17aβ-hydroxy-4,17a-dimethyl-3-oxo-D-homo-5α-androst-16-en-2α-carbonitril.herstellt.

6. Verfahren nach Anspruch 1, dadurch gekennzeichnet, dass man 4,4-Dimethyl-D-homo-androsta-2,5,16-trieno-[2,3-d] isoxazol-17aβ-ol, 4,4,17a-Trimethyl-D-homo-androsta-2,5,16-trieno-[2,3-d]isoxazol-17aβ-ol, 4,4,17a-Trimethyl-D-homo-androsta-2,5-dieno-[2,3-d]isoxazol-17aβ-ol, 4,4-Dimethyl-D-homopregna-2,5,16-trien-20-yno[2,3-d]isoxazol-17aβ-ol, 4,4-Dimethyl-2'H-D-homoandrosta-2,5,16-trieno-[3,2-c]-pyrazol-17aβ-ol, 4,4,17a-Trimethyl-2'H-D-homo-androsta-2,5,16-trieno[3,2-c]pyrazol-17aβ-ol, 17a-Butyl-4,4-dimethyl-2'H-D-homoandrosta-2,5,16-trieno[3,2-c]pyrazol-17aβ-ol, 17a-Butyl-4,4-dimethyl-D-homo-androsta-2,5,16-trieno[2,3-d] isoxazol-17aβ-ol, 4,4-Dimethyl-2'H-D-homo-17aα-pregna-2,5,16-trien-20-yno[3,2-c]pyrazol-17a-ol, 4,5-Dihydro-4',4'-dimethylspiro[furan-2(3H),17'a(beta 1)-D-homoandrosta-[2,5,16]trieno[2,3-d]isoxazol, 4,4,17a-Trimethyl-D-homo-androsta-5,16-dieno-[2,3-d]furazan-17β-ol, 4,4-Dimethyl-2'H-D-homo-androsta-2,5,16-trieno][3,2-c]pyrazol-17aβ-ol und 1'-Acetyl-4,4-dimethyl-1'H-D-homo-17aα-pregna-5,16-dien-20-yno[3,2-c]pyrazol-17a-ol.herstellt.

7. Verfahren nach Anspruch 1, dadurch gekennzeichnet, dass man 17aβ-Hydroxy-4,4,17a-trimethyl-D-homoandrosta-5,16-dien-2,3-dion- 2-oxim und 17aβ-Hydroxy-2-[(hydroxyamino)-methylen]-4,4,17a-trimethyl-D-homoandrosta-5,16-dien-3-on herstellt.

***

Europäisches Patentamt

# EUROPÄISCHER RECHERCHENBERICHT

0116329
Nummer der Anmeldung

EP 84 10 0893

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | US-A-3 966 926 (GORDON O. POTTS)<br>* Insgesamt *<br>--- | 1,10 | C 07 J 63/00<br>C 07 J 71/00<br>A 61 K 31/41<br>A 61 K 31/275<br>A 61 K 31/335 |
| A | US-A-3 980 638 (J.C. BABCOCK)<br>* Insgesamt *<br>--- | 1,10 | |
| A | US-A-4 349 474 (LELAND J. CHINN)<br>* Insgesamt *<br>--- | 1,10 | |
| A | GB-A-2 010 278 (STERLING DRUGS)<br>* Ansprüche; Seiten 1-3 *<br>--- | 1,10 | |
| A | EP-A-0 052 799 (HOFFMANN LA ROCHE)<br>* Ansprüche; Seiten 8-14 *<br>--- | 1,10 | RECHERCHIERTE SACHGEBIETE (Int. Cl. 3) |
| A | US-A-3 917 829 (WALTER VOIGT)<br>* Insgesamt *<br>----- | 1,10 | C 07 J 63/00<br>C 07 J 71/00 |

Der vorliegende Recherchenbericht wurde für alle Patentansprüche erstellt.

| Recherchenort | Abschlußdatum der Recherche | Prüfer |
|---|---|---|
| DEN HAAG | 10-05-1984 | HENRY J.C. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82